# EUROPEAN PATENT SPECIFICATION

(11) **EP 3 541 776 B1**
(45) Date of publication and mention of the grant of the patent: **23.12.2020**
(21) Application number: 17805143.9
(22) Date of filing: 16.11.2017
(51) Int. Cl.: C07C 45/51

(54) **NOVEL PROCESS FOR THE MANUFACTURE OF GAMMA, DELTA-UNSATURATED KETONES**
NEUARTIGES VERFAHREN ZUR HERSTELLUNG VON GAMMA, DELTA-UNGESÄTTIGTEN KETONEN
NOUVEAU PROCÉDÉ DE FABRICATION DE CÉTONES GAMMA, DELTA-INSATURÉES

(30) Priority: 18.11.2016 EP 16199442
(43) Date of publication of application: 25.09.2019
(73) Proprietor: DSM IP Assets B.V., 6411 TE Heerlen (NL)
(72) Inventor: AQUINO, Fabrice, 4303 Kaiseraugst (CH); BONRATH, Werner, 4303 Kaiseraugst (CH); RIEBEL, Peter Hans, 4303 Kaiseraugst (CH)
(74) Representative: Dux, Roland
(86) International application number: PCT/EP2017/079525
(87) International publication number: WO 2018/091623

(56) References cited:
- WO-A2-2010/046199

## Description

WO 2010/046199 A2 discloses a process for the manufacture of gamma,delta-unsaturated ketones using ammonium phosphate as catalyst.

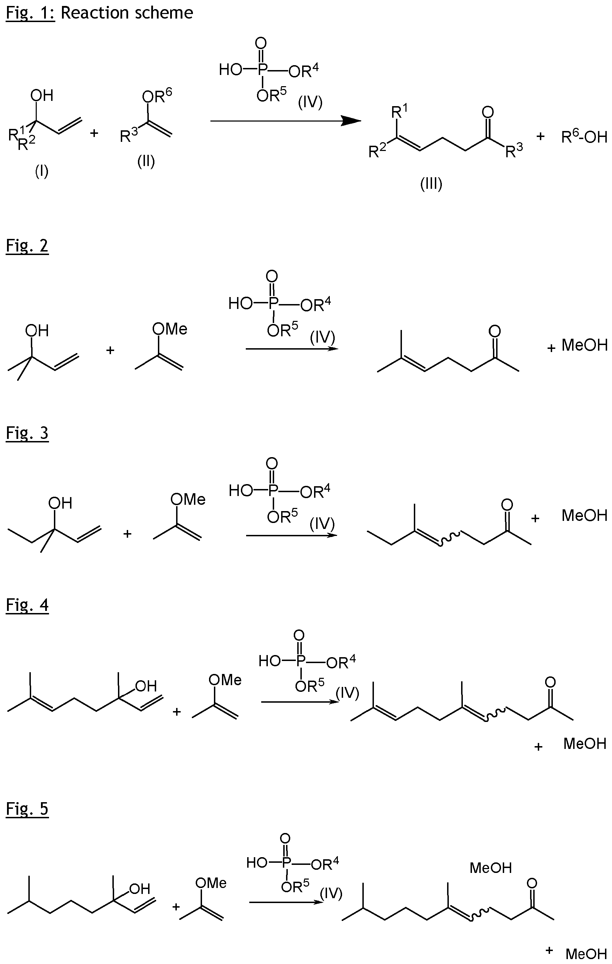

The present invention is directed towards an industrial sustainable process for the manufacture of gamma,delta-unsaturated ketones of formula (III) in the presence of novel catalysts of formula (IV), 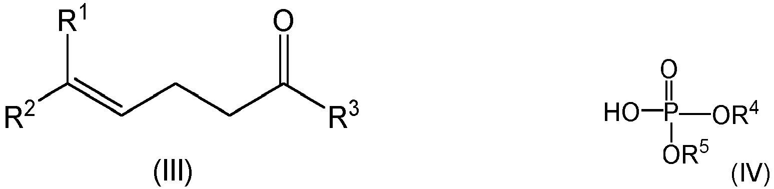
wherein R¹ is either methyl or ethyl, R³ is methyl,
R² is a saturated or unsaturated linear, branched or cyclic hydrocarbyl group with 1 to 46 C atoms, and
R⁴ and R⁵ are independently from each other selected from the group consisting of aralkyl or R⁴ and R⁵ form together 1,1'-biphenyl-2,2'-diyl (see also **Fig. 1**).

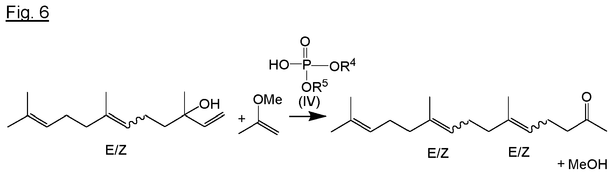

The present invention is especially directed towards an industrial sustainable process for the manufacture of 6-methyl-5-hepten-2-one ("MH"; see **Fig. 2**), 6-methyl-5-octen-2-one ("EH"; see **Fig. 3**), (5*EZ*)-6,10-dimethyl-5,9-undecadien-2-one (*E*)-geranylacetone = "GA"; (*Z*)-nerylacetone = "NA"; see **Fig. 4**), (5*EZ*)-6,10-dimethyl-5-undecen-2-one ((*E*)-dihydrogeranyl-acetone = "DHGA"; (*Z*)-dihydronerylacetone = "DHNA"; see **Fig. 5**) and 6,10,14-trimethyl-5,9,13-pentadeca-trien-2-one (= farnesylacetone; "FA"; see **Fig. 6**).

The preferred catalysts according to the present invention are "DBHP" (dibenzyl hydrogen phosphate) and "BPDHP" (1,1'-biphenyl-2,2'-diyl hydrogen phosphate).

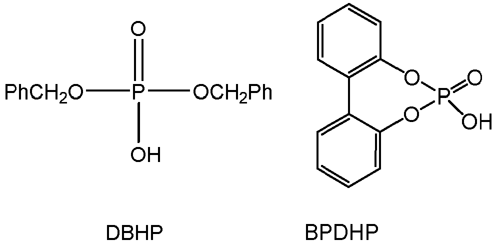

### Detailed description

The present invention is directed towards a process for the manufacture of gamma,delta-unsaturated ketones of the general formula (III) 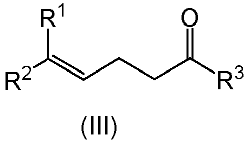 by reacting a tertiary vinyl carbinol of the general formula (I) with an isopropenyl alkyl ether of the general formula (II) in the presence of a catalyst of the general formula (IV), 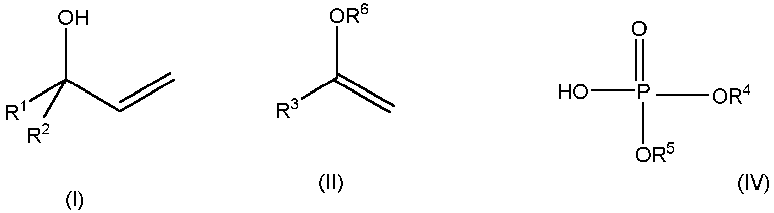
wherein R¹ and R⁶ are independently from each other either methyl or ethyl, R³ is methyl,
R² is a saturated or unsaturated linear, branched or cyclic hydrocarbyl group with 1 to 46 C atoms, and
R⁴ and R⁵ are independently from each other selected from the group consisting of aralkyl or R⁴ and R⁵ form together 1,1'-biphenyl-2,2'-diyl.

### Starting materials

### Compound of the formula (I)

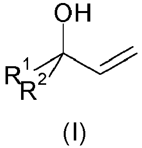

R¹ is either methyl or ethyl, and R² is a saturated or unsaturated linear, branched or cyclic hydrocarbyl group with 1 to 46 C atoms.

Preferred examples of R² are listed in **table 1.**

| **R²** | |
|---|---|
| methyl | CH₃ |
| ethyl | CH₂CH₃ |
| 4-methylpentyl | 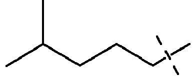 |
| CH₂-prenyl = "homoprenyl" | 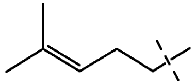 |
| CH₂-geranyl/CH₂-neryl = "homogeranyl"/" homoneryl" | 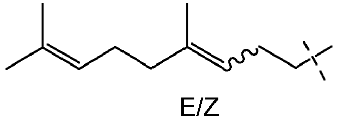 |
| CH₂-farnesyl = "homofarnesyl" | 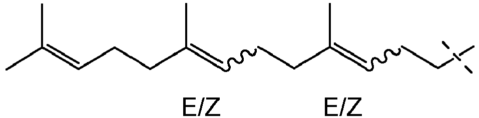 |
| CH₂-hexahydrofarnesyl | 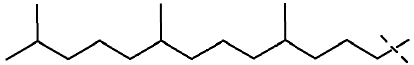 |
| CH₂-solanesyl = "homosolanesyl" | 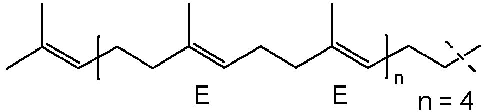 |
| 2-(2',6',6'-trimethylcyclohex-1'-en-1'-yl)ethen-1-yl | 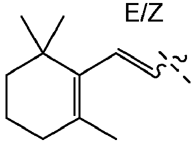 |

The compound of the general formula (I) is preferably selected from the group consisting of 2-methyl-3-buten-2-ol ("MBE"), 3-methyl-1-penten-3-ol ("EBE"), 3,7-dimethyl-1,6-octadien-3-ol (= linalool, "LL"), and (6*E*/6*Z*)-3,7,11-trimethyl-1,6,10-dodecatrien-3-ol (= *E*/*Z*-nerolidol, "*E*/*Z*-NL").

More preferably, the compound of formula (I) is MBE or EBE.

### Compound of the formula (II)

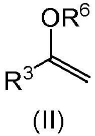

The compound of formula (II) is preferably either isopropenyl methyl ether ("IPM"; R³ = methyl) or isopropenyl ethyl ether ("IPE"; R³ = ethyl), whereby isopropenyl methyl ether is preferred.

### Amounts of compounds of the formula (I) and (II)

The molar ratio of the tertiary vinyl carbinol of the general formula (I) to the isopropenyl alkyl ether of the general formula (II) is preferably ranging from 1:7 to 1:1, more preferably ranging from 1:5 to 1:1.5, most preferably ranging from 1:3.5 to 1:2.

### Catalysts of the formula (IV)

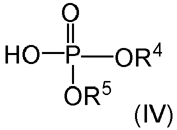

R⁴ and R⁵ are independently from each other selected from the group consisting of aralkyl or R⁴ and R⁵ form together 1,1'-biphenyl-2,2'-diyl.

Preferably R⁴ and R⁵ are either the same aralkyl or they form together 1,1'-biphenyl-2,2' -diyl.

Examples of "aralkyl" are benzyl, 4-methylbenzyl, 2-methylbenzyl, 4-methoxybenzyl, 4-cyanobenzyl, 1-menaphthyl (CH₂-1-naphthyl) and 2-menaphthyl (CH₂-2-naphthyl), whereby benzyl is preferred.

The preferred catalysts according to the present invention are "DBHP" (dibenzyl hydrogen phosphate) and "BPDHP" (biphenyl dihydrogen phosphate).

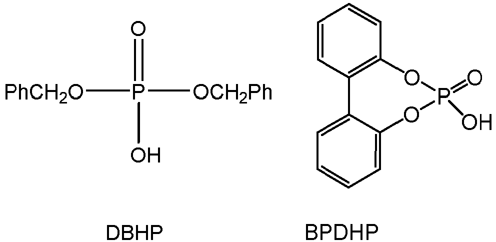

The amount of the catalyst of formula (IV) is preferably ranging from 0.01 - 0.3 mol-%, more preferably ranging from 0.02-0.2 mol-%, most preferably ranging from 0.03-0.1 mol-%, based on the amount of the tertiary vinyl carbinol of the general formula (I).

Advantageously the catalysts of formula (IV) are used as such, i.e. in solid form, and not as solution in an organic solvent such as e.g. acetone.

### Reaction conditions

### Temperature

The reaction is preferably carried out at a temperature ranging from 100 to 170°C, more preferably at a temperature ranging from 110 to 160°C, most preferably at a temperature ranging from 120 to 150°C.

### Pressure

The reaction is preferably carried out at a pressure ranging from 5 to 15 bar, more preferably at a pressure ranging from 8 to 12 bar.

### Solvent

The reaction can be carried out without solvent or in the presence of an organic solvent. Preferably the reaction is carried out without solvent.

Even if the reaction is carried out in the absence of an organic solvent, the starting materials, the compounds of formula (I) and (II), as well as the catalyst of formula (IV) may still be provided in an organic solvent. Thus, there may be an amount of organic solvent up to 10 weight-%, preferably an amount of organic solvent up to 5 weight-%, more preferably an amount of organic solvent up to 3 weight-%, based on the total weight of the reaction mixture, present in the reaction mixture.

If the reaction is carried out in an organic solvent, polar aprotic organic solvents such as aliphatic ketones as e.g. acetone are preferred.

The reaction mixture itself, with or without an organic solvent, is also an object of the present invention. Thus, the present invention is directed towards a reaction mixture comprising a compound of formula (I), a compound of formula (II) and a catalyst of formula (IV), as well as towards a reaction mixture consisting essentially of a compound of formula (I), a compound of formula (II) and a catalyst of formula (IV)

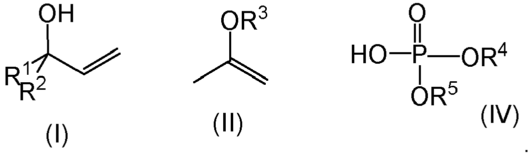

"consisting essentially of" in this context means that the total amount of the compound of formula (I), the compound of formula (II) and the catalyst of formula (IV) sum up to an amount of at least 90 weight-%, preferably at least 95 weight-%, more preferably at least 97 weight-%, based on the total amount of the reaction mixture.

The present invention is also directed towards the use of a catalyst of the formula (IV) 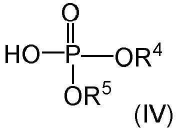 wherein R⁴ and R⁵ are independently from each other selected from the group consisting of aralkyl or R⁴ and R⁵ form together 1,1'-biphenyl-2,2'-diyl,
for the manufacture of gamma,delta-unsaturated ketones of the general formula (III) 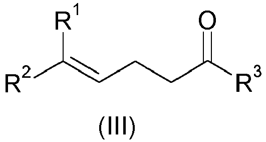 by reacting a tertiary vinyl carbinol of the general formula (I) with an isopropenyl alkyl ether of the general formula (II) 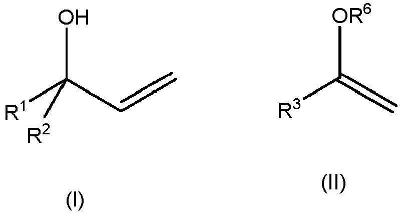
wherein R¹ and R⁶ are independently from each other either methyl or ethyl, R³ is methyl, and
R² is a saturated or unsaturated linear, branched or cyclic hydrocarbyl group with 1 to 46 C atoms, whereby the preferences as given above also apply.

The present invention is now further illustrated by the following nonlimiting examples.

### Examples:

### General procedure

The powdery catalyst (either DBHP or BPDHP in the amounts as given in the tables below) is filled into the reactor as such. 144 g of the starting material (either MBE or EBE or LL or E-NL as given in the tables below) and the amount of IPM ("eq." refers to the molar ratio relative to the other starting material, the compound of formula (I)) as given in the tables below are mixed and poured into the reactor. The closed reactor is then evacuated and released with nitrogen three times. The reaction mixture is stirred and heated up to the temperature as given in the tables below. The experiment is then carried out at isothermal conditions. After the reaction time given in the tables, the reaction mixture is cooled down to 20°C. A sample of the reaction mixture is collected and directly neutralized with sodium acetate. The sample is then analyzed by gas chromatography.

**Table 2: Conditions: 3 eq. IPM/150°C, starting material: MBE, product: MH**

| **Example** | **catalyst** | **Amount of catalyst [mol-%], based on MBE** | **Conversion MBE [%]** | **Yield MH [%]** | **Selectivity** |
|---|---|---|---|---|---|
| 1 | DBHP | 0.1 | 99.7 | 96.6 | 0.97 |

**Table 3: Conditions: 2.1 eq. IPM/150°C, catalyst = BPDHP, starting material = MBE, product = MH**

| **Example** | **2** | **3** |
|---|---|---|
| Amount of catalyst [mol-%] | 0.03 | 0.03 |
| Temperature [°C] | 120 | 150 |
| Conversion MBE [%] | 98.10 | 99.15 |
| Yield MH [%] | 90.00 | 91.43 |
| Selectivity | 0.917 | 0.922 |

**Table 4: Conditions: 2.1 eq. IPM/150°C, catalyst = BPDHP, starting material = EBE, product = EH**

| **Example** | **4** |
|---|---|
| Amount of catalyst [mol-%] | 0.03 |
| Temperature [°C] | 120 |
| Conversion EBE [%] | 96.9 |
| Yield EH [%] | 93.0 |
| Selectivity | 0.96 |

**Table 5: Conditions: 3.8 eq. IPM/150°C, catalyst = BPDHP, starting material = LL, product = GA**

| **Example** | **5** |
|---|---|
| Amount of catalyst [mol-%] | 0.1 |
| Temperature [°C] | 160 |
| Conversion LL [%] | 99.3 |
| Yield GA [%] | 91.6 |
| Selectivity | 0.92 |

**Table 6: Conditions: 3.8 eq. IPM/150°C, catalyst = BPDHP, starting material = E-NL, product = FA**

| Example | 6 |
|---|---|
| Amount of catalyst [mol-%] | 0.1 |
| Temperature [°C] | 160 |
| Conversion E-NL [%] | 99.5 |
| Yield FA [%] | 90.3 |
| Selectivity | 0.91 |

## Claims

1. A process for the manufacture of gamma,delta-unsaturated ketones of the general formula (III) 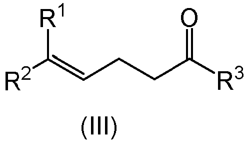 by reacting a tertiary vinyl carbinol of the general formula (I) with an isopropenyl alkyl ether of the general formula (II) in the presence of a catalyst of the general formula (IV), 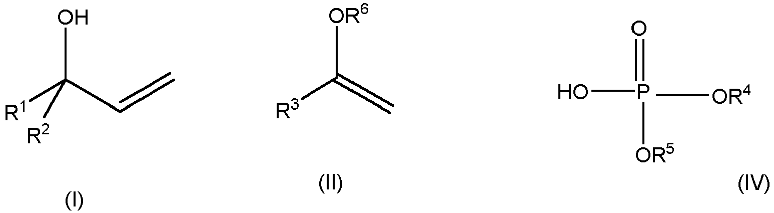
wherein R¹ and R⁶ are independently from each other either methyl or ethyl, R³ is methyl,
R² is a saturated or unsaturated linear, branched or cyclic hydrocarbyl group with 1 to 46 C atoms, and
R⁴ and R⁵ are independently from each other selected from the group consisting of aralkyl or R⁴ and R⁵ form together 1,1'-biphenyl-2,2'-diyl.

2. The process according to claim 1, wherein R⁴ and R⁵ are both benzyl or form together 1,1'-biphenyl-2,2'-diyl.

3. The process according to any one or more of the preceding claims, wherein the reaction is carried out at a temperature ranging from 100 to 170°C, preferably at a temperature ranging from 110 to 160°C, more preferably at a temperature ranging from 120 to 150°C.

4. The process according to any one or more of the preceding claims, wherein the reaction is carried out at a pressure ranging from 5 to 15 bar, preferably at a pressure ranging from 8 to 12 bar.

5. The process according to any one or more of the preceding claims, wherein the molar ratio of the tertiary vinyl carbinol of the general formula (I) to the isopropenyl alkyl ether of the general formula (II) is ranging from 1:7 to 1:1, preferably ranging from 1:5 to 1:1.5, more preferably ranging from 1:3.5 to 1:2.

6. The process according to any one or more of the preceding claims, wherein the amount of the catalyst is ranging from 0.01-0.3 mol-%, preferred ranging from 0.02-0.1 mol-%, more preferred ranging from 0.02-0.07 mol-%, based on the amount of the tertiary vinyl carbinol of the general formula (I).

7. The process according to any one or more of the preceding claims, wherein the compound of the general formula (I) is selected from the group consisting of 2-methyl-3-buten-2-ol, 3-methyl-1-penten-3-ol, 3,7-dimethyl-1,6-octadien-3-ol and (6*E*/6*Z*)-3,7,11-trimethyl-1,6,10-dodecatrien-3-ol, preferably wherein the compound of the general formula (I) is 2-methyl-3-buten-2-ol.

8. Use of a catalyst of the formula (IV) 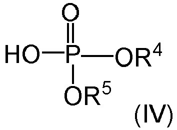
wherein R⁴ and R⁵ are independently from each other selected from the group consisting of aralkyl or R⁴ and R⁵ form together 1,1'-biphenyl-2,2'-diyl,
for the manufacture of gamma,delta-unsaturated ketones of the general formula (III) 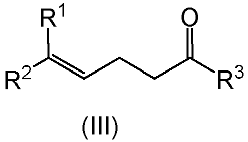 by reacting a tertiary vinyl carbinol of the general formula (I) with an isopropenyl alkyl ether of the general formula (II) 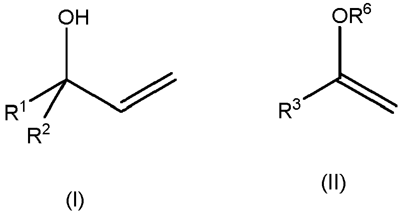
wherein R¹ and R⁶ are independently from each other either methyl or ethyl, R³ is methyl, and
R² is a saturated or unsaturated linear, branched or cyclic hydrocarbyl group with 1 to 46 C atoms.

9. A reaction mixture comprising a compound of formula (I), a compound of formula (II) and a catalyst of formula (IV) 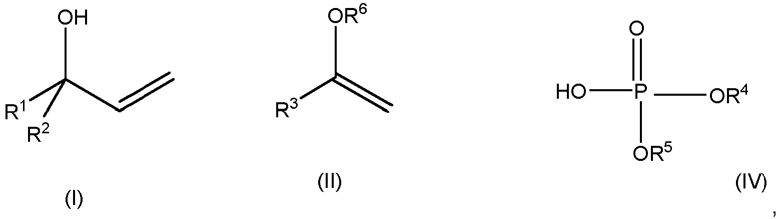
wherein R¹ and R⁶ are independently from each other either methyl or ethyl, R³ is methyl, and
R² is a saturated or unsaturated linear, branched or cyclic hydrocarbyl group with 1 to 46 C atoms, and R⁴ and R⁵ are independently from each other selected from the group consisting of aralkyl or R⁴ and R⁵ form together 1,1'-biphenyl-2,2'-diyl.

10. A reaction mixture consisting essentially of a compound of formula (I), a compound of formula (II) and a catalyst of formula (IV) 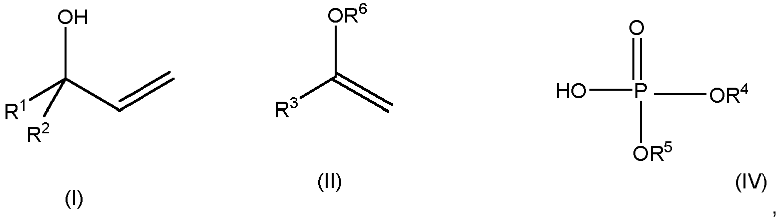
wherein R¹ and R⁶ are independently from each other either methyl or ethyl, R³ is methyl, and
R² is a saturated or unsaturated linear, branched or cyclic hydrocarbyl group with 1 to 46 C atoms, and R⁴ and R⁵ are independently from each other selected from the group consisting of aralkyl or R⁴ and R⁵ form together 1,1'-biphenyl-2,2'-diyl.

## Patentansprüche

1. Verfahren zur Herstellung von gamma, deltaungesättigten Ketonen der allgemeinen Formel (III) 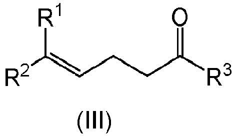 durch Umsetzen eines tertiären Vinylcarbinols der allgemeinen Formel (I) mit einem Isopropenylalkylether der allgemeinen Formel (II) in Gegenwart eines Katalysators der allgemeinen Formel (IV), 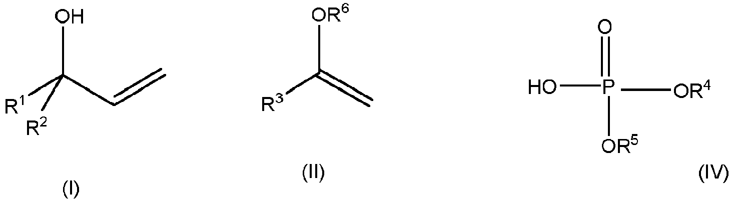
wobei R¹ und R⁶ unabhängig voneinander entweder für Methyl oder Ethyl stehen, R³ für Methyl steht,
R² für eine gesättigte oder ungesättigte lineare, verzweigte oder cyclische Hydrocarbylgruppe mit 1 bis 46 C-Atomen steht und
R⁴ und R⁵ unabhängig voneinander aus der Gruppe bestehend aus Aralkyl ausgewählt sind oder R⁴ und R⁵ zusammen 1,1'-Biphenyl-2,2'-diyl bilden.

2. Verfahren nach Anspruch 1, wobei R⁴ und R⁵ beide für Benzyl stehen oder zusammen 1,1'-Biphenyl-2,2'-diyl bilden.

3. Verfahren nach einem oder mehreren der vorhergehenden Ansprüche, wobei die Umsetzung bei einer Temperatur im Bereich von 100 bis 170 °C, vorzugsweise bei einer Temperatur im Bereich von 110 bis 160 °C, weiter bevorzugt bei einer Temperatur im Bereich von 120 bis 150 °C, durchgeführt wird.

4. Verfahren nach einem oder mehreren der vorhergehenden Ansprüche, wobei die Umsetzung bei einem Druck im Bereich von 5 bis 15 bar, vorzugsweise bei einem Druck im Bereich von 8 bis 12 bar, durchgeführt wird.

5. Verfahren nach einem oder mehreren der vorhergehenden Ansprüche, wobei das Molverhältnis von tertiärem Vinylcarbinol der allgemeinen Formel (I) zu Isopropenylalkylether der allgemeinen Formel (II) im Bereich von 1:7 bis 1:1, vorzugsweise im Bereich von 1:5 bis 1:1,5, weiter bevorzugt im Bereich von 1:3,5 bis 1:2, liegt.

6. Verfahren nach einem oder mehreren der vorhergehenden Ansprüche, wobei die Menge des Katalysators im Bereich von 0,01-0,3 Mol-%, vorzugsweise im Bereich von 0,02-0,1 Mol-%, weiter bevorzugt im Bereich von 0,02-0,07 Mol-%, bezogen auf die Menge des tertiären Vinylcarbinols der allgemeinen Formel (I), liegt.

7. Verfahren nach einem der vorhergehenden Ansprüche, wobei die Verbindung der allgemeinen Formel (I) aus der Gruppe bestehend aus 2-Methyl-3-buten-2-ol, 3-Methyl-1-penten-3-ol, 3,7-Dimethyl-1,6-octadien-3-ol und (6*E*/6*Z*)-3,7,11-Trimethyl-1,6,10-dodecatrien-3-ol ausgewählt ist, vorzugsweise wobei es sich bei der Verbindung der allgemeinen Formel (I) um 2-Methyl-3-buten-2-ol handelt.

8. Verwendung eines Katalysators der Formel (IV) 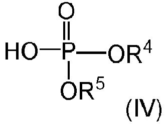 wobei R⁴ und R⁵ unabhängig voneinander aus der Gruppe bestehend aus Aralkyl ausgewählt sind oder R⁴ und R⁵ zusammen 1,1'-Biphenyl-2,2'-diyl bilden, zur Herstellung von gamma,delta-ungesättigten Ketonen der allgemeinen Formel (III) 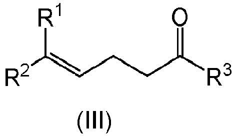 durch Umsetzen eines tertiären Vinylcarbinols der allgemeinen Formel (I) mit einem Isopropenylalkylether der allgemeinen Formel (II) 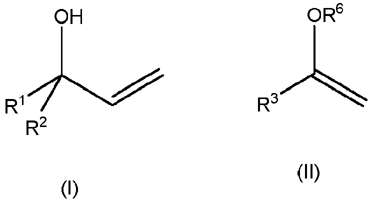 wobei R¹ und R⁶ unabhängig voneinander entweder für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht.

9. Reaktionsmischung, umfassend eine Verbindung der Formel (I), eine Verbindung der Formel (II) und einen Katalysator der Formel (IV) 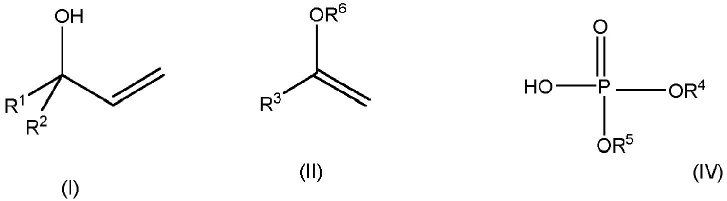
wobei R¹ und R⁶ unabhängig voneinander entweder für Methyl oder Ethyl stehen, R³ für Methyl steht und
R² für eine gesättigte oder ungesättigte lineare, verzweigte oder cyclische Hydrocarbylgruppe mit 1 bis 46 C-Atomen steht und R⁴ und R⁵ unabhängig voneinander aus der Gruppe bestehend aus Aralkyl ausgewählt sind oder R⁴ und R⁵ zusammen 1,1'-Biphenyl-2,2'-diyl bilden.

10. Reaktionsmischung, im Wesentlichen bestehend aus einer Verbindung der Formel (I), einer Verbindung der Formel (II) und einem Katalysator der Formel (IV) 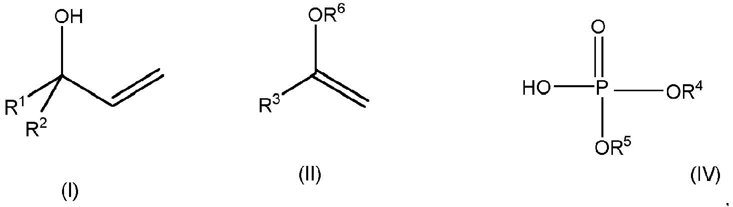 wobei R¹ und R⁶ unabhängig voneinander entweder für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht und R⁴ und R⁵ unabhängig voneinander aus der Gruppe bestehend aus Aralkyl ausgewählt sind oder R⁴ und R⁵ zusammen 1,1'-Biphenyl-2,2'-diyl bilden.

## Revendications

1. Procédé de fabrication de cétones gamma,delta-insaturées de formule générale (III) 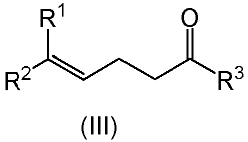 par réaction d'un vinylcarbinol tertiaire de formule générale (I) avec un éther d'isopropényle d'alkyle de formule générale (II) en présence d'un catalyseur de formule générale (IV), 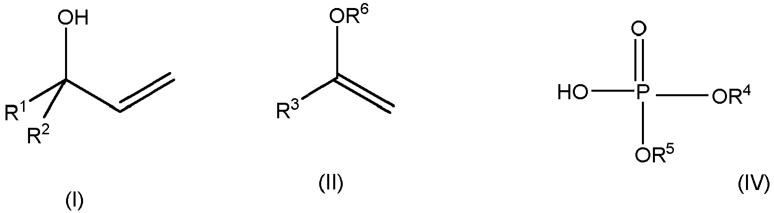
dans lequel R¹ et R⁶ sont, indépendamment l'un de l'autre, méthyle ou éthyle, R³ est méthyle,
R² est un groupe hydrocarbyle linéaire, ramifié ou cyclique, saturé ou insaturé avec 1 à 46 atomes C, et
R⁴ et R⁵ sont, indépendamment l'un de l'autre, choisis dans le groupe constitué d'aralkyle ou R⁴ et R⁵ forment conjointement 1,1'-biphényl-2,2'-diyle.

2. Procédé selon la revendication 1, dans lequel R⁴ et R⁵ sont tous deux benzyle ou forment conjointement 1,1'-biphényl-2,2'-diyle.

3. Procédé selon l'une ou plusieurs quelconques des revendications précédentes, dans lequel la réaction est conduite à une température dans la plage de 100 à 170 °C, de préférence à une température dans la plage de 110 à 160 °C, plus préférablement à une température dans la plage de 120 à 150 °C.

4. Procédé selon l'une ou plusieurs quelconques des revendications précédentes, dans lequel la réaction est conduite à une pression dans la plage de 5 à 15 bar, de préférence à une pression dans la plage de 8 à 12 bar.

5. Procédé selon l'une ou plusieurs quelconques des revendications précédentes, dans lequel le rapport molaire du vinylcarbinol tertiaire de formule générale (I) à l'éther d'isopropényle d'alkyle de formule générale (II) est dans la plage de 1:7 à 1:1, de préférence dans la plage de 1:5 à 1:1,5, plus préférablement dans la plage de 1:3,5 à 1:2.

6. Procédé selon l'une ou plusieurs quelconques des revendications précédentes, dans lequel la quantité du catalyseur est dans la plage de 0,01 à 0,3 % en moles, de préférence dans la plage de 0,02 à 0,1 % en moles, plus préférablement dans la plage de 0,02 à 0,07 % en moles, sur la base de la quantité du vinylcarbinol tertiaire de formule générale (I).

7. Procédé selon l'une ou plusieurs quelconques des revendications précédentes, dans lequel le composé de formule générale (I) est choisi dans le groupe constitué des 2-méthyl-3-butén-2-ol, 3-méthyl-1-pentén-3-ol, 3,7-diméthyl-1,6-octadién-3-ol et (6E/6Z)-3,7,11-triméthyl-1,6,10-dodécatrién-3-ol, de préférence dans lequel le composé de formule générale (I) est le 2-méthyl-3-butén-2-ol.

8. Utilisation d'un catalyseur de formule (IV) 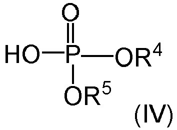
dans lequel R⁴ et R⁵ sont, indépendamment l'un de l'autre, choisis dans le groupe constitué d'aralkyle ou R⁴ et R⁵ forment conjointement 1,1'-biphényl-2,2'-diyle,
pour la fabrication de cétones gamma,delta-insaturées de formule générale (III) 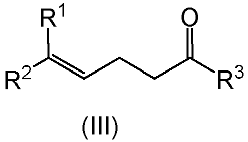 par réaction d'un vinylcarbinol tertiaire de formule générale (I) avec un éther d'isopropényle d'alkyle de formule générale (II) 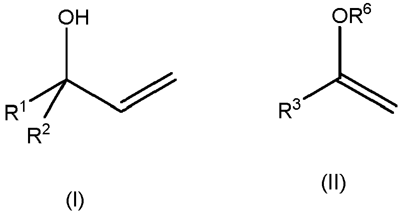
dans lequel R¹ et R⁶ sont, indépendamment l'un de l'autre, méthyle ou éthyle, R³ est méthyle, et
R² est un groupe hydrocarbyle linéaire, ramifié ou cyclique, saturé ou insaturé avec 1 à 46 atomes C.

9. Mélange de réaction comprenant un composé de formule (I), un composé de formule (II) et un catalyseur de formule (IV) 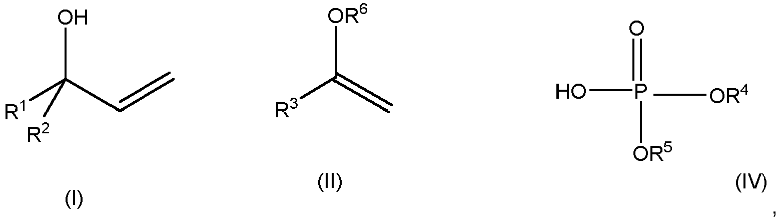
dans lesquels R¹ et R⁶ sont, indépendamment l'un de l'autre, méthyle ou éthyle, R³ est méthyle, et
R² est un groupe hydrocarbyle linéaire, ramifié ou cyclique, saturé ou insaturé avec 1 à 46 atomes C, et R⁴ et R⁵ sont, indépendamment l'un de l'autre, choisis dans le groupe constitué d'aralkyle ou R⁴ et R⁵ forment conjointement 1,1'-biphényl-2,2'-diyle.

10. Mélange de réaction essentiellement constitué d'un composé de formule (I), d'un composé de formule (II) et d'un catalyseur de formule (IV) 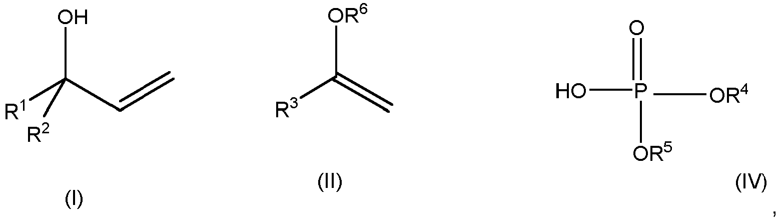
dans lesquels R¹ et R⁶ sont, indépendamment l'un de l'autre, méthyle ou éthyle, R³ est méthyle, et
R² est un groupe hydrocarbyle linéaire, ramifié ou cyclique, saturé ou insaturé avec 1 à 46 atomes C, et R⁴ et R⁵ sont, indépendamment l'un de l'autre, choisis dans le groupe constitué d'aralkyle ou R⁴ et R⁵ forment conjointement 1,1'-biphényl-2,2'-diyle.
